(19) Europäisches Patentamt
European Patent Office
Office européen des brevets

(11) Veröffentlichungsnummer: **0 000 723**
**B1**

(12) # EUROPÄISCHE PATENTSCHRIFT

(21) Anmeldenummer: **78100486.6**

(22) Anmeldetag: **24.07.78**

(51) Int. Cl.³: **C 08 G 18/77,**
C 08 G 18/71,
C 07 C 143/68,
C 08 G 18/50,
C 08 G 18/46

(54) **Urethan-aryl-sulfonsäure-hydroxyalkylester-Gruppen aufweisende Polyhydroxyverbindungen, Verfahren zu deren Herstellung und deren Verwendung als Reaktionskomponente bei der Herstellung von Polyurethankunststoffen**

(30) Priorität: **03.08.77 DE 2735032**

(43) Veröffentlichungstag der Anmeldung:
**21.02.79 Patentblatt 79/04**

(45) Bekanntmachung des Hinweises auf die Patenterteilung:
**07.01.81 Patentblatt 81/01**

(84) Benannte Vertragsstaaten:
**BE DE FR GB NL**

(56) Entgegenhaltungen:
**FR - A - 2 264 010**
**FR - A - 2 370 064**

(73) Patentinhaber: **BAYER AG**
**Zentralbereich Patente, Marken und Lizenzen**
**D - 5090 Leverkusen 1, Bayerwerk (DE)**

(72) Erfinder: **Dieterich, Dieter, Dr.**
**Ludwig-Girtler-Strasse 1**
**D - 5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 723 B1

Urethan-aryl-sulfonsäure-hydroxyalkylester-Gruppen aufweisende Polyhydroxyverbindungen, Verfahren zu deren Herstellung und deren Verwendung als Reaktionskomponente bei der Herstellung von Polyurethankunststoffen

Es ist bekannt, zur Herstellung von Polyurethanen Polyisocyanate mit Verbindungen umzusetzen welche zwei bis sechs OH-Gruppen enthalten und ein Molekulargewicht zwischen 62 und etwa 10.000 aufweisen. Zu diesen Polyhydroxyverbindungen zählen beispielsweise:

di- und höherfunktionelle Alkohole, wie z.B. Äthylenglykol, Diäthylenglykol, Hexandiol, Glycerin, Trimethylolpropan sowie höhermolekulare Polyäther, Polythioäther, Polyester, Polyacetale. Die höhermolekularen Polyhydroxyverbindungen werden in bekannter Weise aus niedermolekulares Bausteinen hergestellt.

In diesen Polyhydroxyverbindungen sind im allgemeinen die verschiedenen OH-Funktionen gleichwertig in Bezug auf Reaktivität und Entfernung von einem ggf. vorhandenen Verzweigungs-Zentrum. Ausnahmen sind niedermolekulare Alkohole, welche primäre und sekundäre Hydroxygruppen aufweisen, wie beispielsweise Glycerin. Bei höhermolekularen Polyäthern und Polyestern sind zwar haufig ebenfalls sowohl primäre als auch sekundäre OH-Gruppen zugegen, jedoch ist deren Verteilung statistisch, so daß es nicht möglich ist, aufgrund dieses Reaktivitätsunterschiedes Polymere mit definierter Struktur aufzubauen. Auch die Kettenlängenverteilungen bei verzweigten Polyäthern und Polyestern gehorchen den Gesetzen der Statistik.

Es ist ebenfalls bekannt, die o.g. Polyhydroxyverbindungen durch eine molar unterschüssige Menge eines Polysiocyanats zu OH-Präpolymeren zu verlängern. Dabei tritt im Falle trifunktionaller Isocyanate zwar eine Verzweigung ein, die Reaktivität der OH-Gruppen sowie die Kettenlängenverteilung sind aber wiederum statistisch.

eine gesonderte Herstellung von OH-Präpolymeren zur nachfolgenden Herstellung von Polyurethanen ist im übrigen meist nicht sinnvoll, da durch Herstellung nach dem one-shot-Verfahren oder über NCO-Präpolymere dieselben Polyurethan-Strukturen entstehen.

Zur Herstellung von Polyurethanen und insbesondere zur Herstellung räumlich vernetzter Polyurethane sind nun Polyhydroxyverbindungen erwünscht, welche in definierter Weise OH-Gruppen verschiedener Reaktivität und Kettenzweige unterschiedlicher Länge aufweisen. Es wäre beispielsweise vorteilhaft, über trifunktionelle Polyhydroxyverbindungen zu verfügen, welche 2 OH-Gruppen hoher Reaktivität an den Enden der Hauptkette sowie eine OH-Funktion verminderter Reaktivität an einer möglichst kurzen Seitenkette aufweisen würden, weil von einer derartigen Struktur ein Polymer mit besonders günstigen mechanischen Eigenschaften zu erwarten ist. Weiterhin ist erwünscht, Polyhydroxyverbindungen einsetzen zu können, welche Polyurethane mit verbessertem Brandverhalten liefern. Schließlich sind OH-Präpolymere erwünscht, welche beim hydrolytischen Abbau keine toxikologisch bedenklichen aromatischen Diamine liefern.

Reaktion der Umsetzungsprodukte mit Oxiranen oder Oxetanen neuartige Polyhydroxyverbindungen höherer Funktionalität erhalten werden. Dabei befindet sich die aus der Reaktion der Sulfonsäuregruppe mit dem cyclischen Ather entstandene OH-Gruppe an einer kurzen Seitenkette.

Weiterhin wurde gefunden, daß durch Umsetzung üblicher Polyhydroxyverbindungen mit äquivalenten Mengen an Monoisocyanat- mono- oder Polysulfonsäuren und anschließende Reaktion der Umsetzungsprodukte mit Oxiranen oder Oxetanen neuartige Polyhydroxy-Verbindungen mit veränderter Funktionalität und/oder Reaktivität erhalten werden.

Gegenstand der vorliegenden Erfindung sind somit mindestens zwei Hydroxygruppen und mindestens eine Sulfonsäureester-Gruppe aufweisende Verbindungen vom durchschnittlichen Molekulargewicht 300 bis 12000, in denen mindestens eine Hydroxygruppe in Form eines Urethan-aryl-sulfonsäure-hydroxy-alkylesters vorliegt.

Erfindungsgemäß bevorzugt sind dabei Verbindungen, welche ein durchschnittliches Molekulargewicht von 300 bis 12000 aufweisen, gekennzeichnet durch mindestens eine OH-funktionelle Langkette, welche 6 bis 400 Kettenglieder, vorzugsweise 20 bis 300 Kettenglieder, enthält und mindestens eine über einen Sulfonsäureester-Rest mit einer Verzweigungsstelle verbundene OH-funktionelle Kurzkette, welche 2 bis 3 Kettenglieder enthält, sowie mindestens einen, mindestens trifunktionellen Arylrest als Verzweigungsstelle.

Die erfindungsgemäßen Verbindungen enthalten bevorzugt mindestens eine Struktureinheit der allgemeinen Formel:

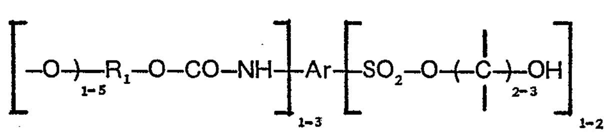

in der

$R_1$ einen 2- bis 6-wertigen Rest eines Polyols, z.B. eines Polyesters, Polyäthers, Polythioäthers oder Polyesteramids und

Ar einen mehrwertigen Rest eines aromatischen Isocyanats darstellt,

insbesondere mindestens eine Struktureinheit der allgemeinen Formel

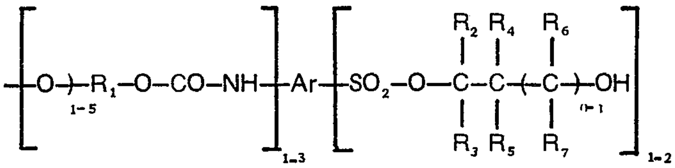

in der

$R_1$ und Ar die schon genannten Bedeutungen haben und

$R_2$, $R_4$ H, $C_1$—$C_8$-Alkyl, $C_6$—$C_{14}$-Aryl, Rest eines Epoxids, vorzugsweise —$CH_2$—O—$R_8$, —$CH_2$—X, $CH_2$—O—CO—$R_9$, weitere Epoxidgruppen enthaltender aliphatischer $C_1$—$C_8$-Alkylrest,

$R_3$, $R_5$, $R_6$, $R_7$, H, $C_1$—$C_8$-Alkyl, $C_6$—$H_{14}$-Aryl und

$R_8$, $R_9$ $C_1$—$C_8$-Alkyl, $C_6$—$H_{14}$-Aryl und X OH, Cl, Br, CN

bedeuten.

Erfindungsgemäß sind Verbindungen folgender allgemeiner Formeln bevorzugt:

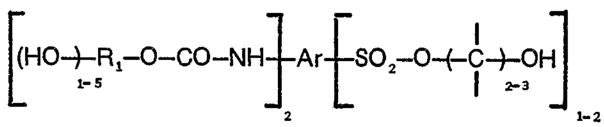

insbesondere

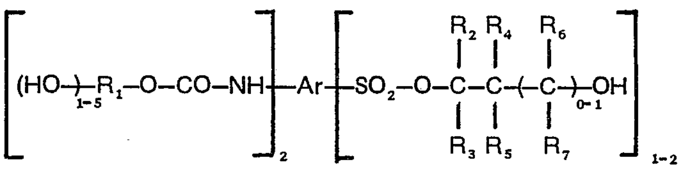

und

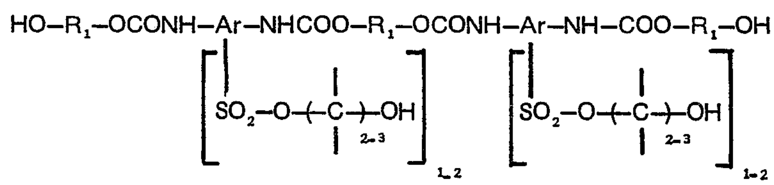

insbesondere

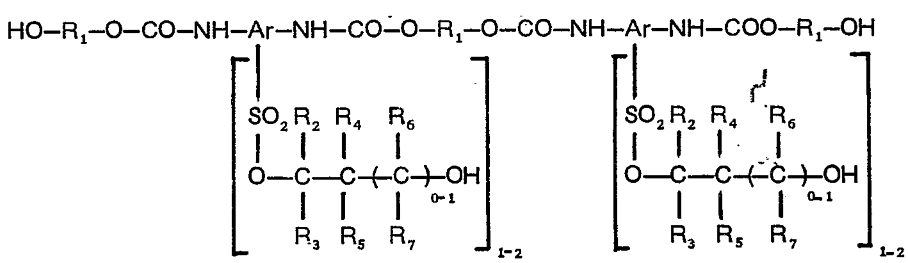

in denen

$R_1$, $R_2$, $R_3$, $R_4$, $R_5$, $R_6$, $R_7$ und Ar die schon genannte Bedeutung aufweisen.

Gegenstand der vorliegenden Erfindung ist auch ein Verfahren zur Herstellung von mindestens zwei Hydroxylgruppen und mindestens eine Sulfonsäureestergruppe aufweisenden Verbindungen, vom durchschnittlichen Molekulargewicht 300 bis 12000, in denen mindestens eine Hydroxylgruppe in Form eines Urethan-aryl-sulfonsäure-hydroxyalkylesters vorliegt, dadurch gekennzeichnet, daß mindestens zwei Hydroxygruppen aufweisende Verbindungen vom Molekulargewicht 62 bis 10000 bei 0—190°C mit Isocyanatosulfonsäure und anschließend mit Oxiranen und/oder Oxetanen umgesetzt werden, wobei das Äquivalent-Verhältnis der Gesamtmenge an Isocyanatgruppen (einschließlich der gegebenenfalls in dimerisierter Form vorhandenen Isocyanatgruppen) zu Sulfonsäuregruppen 0,5 bis 50, das Äquivalent-Verhältnis aus der Summe der Hydroxylgruppen der mindestens zwei Hydroxyl-

gruppen aufweisenden Verbindungen und der Sulfonsäuregruppen zu NCO-Gruppen 1,5 bis 30 und das Äquivalent-Verhältnis der Oxiran- bzw. Oxetan-Gruppen zu Sulfonsäuregruppen 0,2 bis 5 beträgt.

Gegenstand der vorliegenden Erfindung ist schließlich auch die Verwendung der erfindungsgemäßen Verbindungen als Reaktionskomponente für Polyisocyanate zur Herstellung von Polyadditionsprodukten oder Polykondensationsprodukten.

Die neuen erfindungsgemäßen Verbindungen besitzen gegenüber den bisher bekannten Polyhydroxyverbindungen eine Reihe von vorteilhaften Eigenschaften:

1. Sie besitzen stark polaren Charakter, außerordentlich niedrigen Dampfdruck und sind hervorragend verträglich mit einer Vielzahl polarer und apolarer Medien und Reaktionspartnern.

2. In Abhängigkeit von der chemischen Konstitution der eingesetzten Oxirans bzw. Oxetans läßt sich die Reaktivität der über eine kurze Seitenkette an die Verzweigungsstelle gebundenen OH-Gruppe wunschgemäß steuern. Die Reaktivität dieser OH-Gruppe kann höher, nahezu gleich oder auch geringer sein als die der über die Polyhydroxyverbindung eingeführten OH-Gruppe.

3. Die Funktionalität der als Ausgangsmaterial eingesetzten Polyhydroxyverbindungen läßt sich in Abhängigkeit von der Menge an eingesetzter Isocyanatosulfonsäure wunschgemäß erhöhen, z.B. von 2 auf 2,1 oder z.B. auch auf 3 oder 4.

4. In Abhängigkeit von Natur und Menge des verwendeten Oxirans oder Oxetans läßt sich die Hydrophilie und die Acidität der Produkte in weiten Grenzen steuern. Bei vollständiger Umsetzung der Sulfonsäure-Gruppen mit Oxiranen oder Oxetanen erhält man weitgehend hydrophobe Polyhydroxyverbindungen.

5. Der hydrolytische Abbau der Produkte führt zu toxikologisch unbedenklichen Polyaminosulfonsäuren.

6. Die Verwendung der erfindungsgemäßen Verbindungen beispielsweise bei der Herstellung von Polyurethanen führt zu Polymeren mit verbessertem Brandverhalten.

Die erfindungsgemäß erhaltenen Produkte bzw. die beim Aufbau von polyurethanen aus diesen Produkten erhaltenen Kettensegmente sind auf andere Weise nicht ohne weiteres erhältlich, da die direkte Umsetzung von Isocyanatoarylsulfonsäuren oder daraus hergestellten NCO-Präpolymeren mit Oxiranen bzw. Oxetanen andere Produkte bzw. andersartig gebaute Kettensegmente liefert. Bevorzugt enthalten die erfindungsgemäßen Verbindungen mindestens ein Segement, das einen 2- bis 6-wertigen Rest eines Polyäthers, Polythioäthers, Polyesters oder Polyesteramids darstellt.

Bei der Durchführung des erfindungsgemäßen Verfahrens erfolgt normalerweise in einem ersten Reaktionsschritt zunächst eine Addition eines Teils der OH-Gruppen der als Ausgangsmaterial verwendeten Polyhydroxyverbindungen mit den NCO-Gruppen sowie gegebenenfalls vorhandenen Uretdion-Gruppen der Isocyanatoarylsulfonsäure unter Ausbildung von höhermolekularen neuen Polyhydroxyverbindungen, welche zunächst anteilig Urethan-Gruppen und eine oder mehrere freie Sulfonsäure-Gruppen enthalten. Die Sulfonsäure-Gruppe wird anschließend durch das zugesetzte Oxiran bzw. Oxetan verestert, wodurch Hydroxyalkylsulfonsäureester-Gruppen entstehen.

Beim erfindungsgemäßen Verfahren können alle in der Polyurethanchemie üblicherweise verwendeten Verbindungen mit mindestens zwei Hydroxylgruppen und einem Molekulargewicht von 62 bis 10 000 als Ausgangsmaterial eingesetzt werden. Beispiele hierfür sind niedermolekulare Glykole, Polyester, Polyäther, Polyesteramide, OH-Gruppen aufweisende Oligomere und Polymerisate, beispielsweise auf Basis Butadien, sowie durch Vinylmonomere gepfropfte Polyäther oder auch solche Polyhydroxylverbindungen, welche andere Polymere, wie z.B. Polyharnstoffe, Harnstoffharze, Polyhydrazodicarbonamide oder Vinylpolymerisate dispergiert enthalten. Geeignete Hydroxylgruppen aufweisende Verbindungen dieser Art werden beispielsweise in den deutschen Auslegeschriften 1 152 536, 1 168 075 und 1 260 142, den deutschen Offenlegungsschriften 2 324 134, 2 423 984, 2 512 385, 2 513 815, 2 550 796, 2 550 797, 2 550 833, 2 550 662 und 2 550 860 sowie den US-Patentschriften 3 383 351, 3 304 273, 3 523 093, 3 110 695 und 3 869 413 beschrieben.

Beim erfindungsgemäßen Verfahren können als Isocyanatoarylsulfonsäuren die Sulfonierungsprodukte aller bekannten aromatischen Polyisocyanate eingesetzt werden. Beispiele für derartige in Form ihrer Sulfonierungsprodukte beim erfindungsgemäßen Verfahren einsetzbare aromatische Polyisocyanate sind:

4,4' - Stilbendiisocyanat, 4,4' - Dibenzyldiisocyanat, 3,3' - bzw. 2,2' - dimethyl - 4,4' - diisocyanato - diphenylmethan, 2,3,2',5' - Tetramethyl - 4,4' - diisocyanato - diphenylmethan, 3,3' - Dimethoxy - 4,4' - diisocyanato - diphenylmethan, 3,3' - Dichlor - 4,4' - diisocyanato - diphenylmethan, 4,4' - Diisocyanato - diphenylcyclohexylmethan, 4,4' - Diisocyanato - benzophenon, 4,4' - Diisocyanato diphenylsulfon, 4,4' Diisocyanato-diphenyläther, 4,4' - Diisocyanato - 3,3' - dibrom - diphenylmethan, 4,4'-Diisocyanato - 3,3'-diäthyl - diphenylmethan, 4,4' - Diisocyanato - diphenyl - äthylen - (1,2), 4,4' - Diisocyanato - diphenyl - sulfid, 1,3- und 1,4 - Phenylendiisocyanat, 2,4- und 2,6 - Toluylendiisocyanat sowie bliebige Gemische dieser Isomeren, Diphenylmethan - 2,4' - und/oder -4,4' - diisocyanat, Naphthylen-1,5 - diisocyanat, Triphenylmethan - 4,4' -. 4'' - triisocyanat, Polyphenyl - polymethylen - polyisocyanate, wie sie durch Anilin - Formaldehyd - Kondensation und anschließende Phosgenierung erhalten und z.B. in den britischen Patentschriften 874 430 und 848 671 beschrieben werden, Carbodiimidgruppen aufweisende Polyisocyanate, wie sie in der deutschen Patentschrift 1 092 007 beschrieben werden, Diisocyanate, wie sie in der amerikanischen Patentschrift 3 492 330 beschrieben werden, Allophanatgruppen aufweisende Polyisocyanate, wie sie z.B. in der britischen Patentschrift 994 890, der belgischen Patentschrift 761 626 und der veröffentlichten holländischen Patentanmeldung 7 102 524 beschrieben werden, Isocyanuratgruppen aufweisende Polyisocyanate, wie sie z.B. in der deutschen Patentschriften 1 022 789, 1 222 067 und 1 027 394, sowie in den deutschen Offenlegungsschriften 1 929 034 und 2 004 048 beschriben werden, acylierte Harnstoffgruppen aufweisende Polyisocyanate gemäß der deutschen Patentschrift 1 230 778, Biuretgruppen aufweisende Polyisocyanate, wie sie z.B. in der deutschen Patentschrift 1 101 394, in der britischen Patentschrift 889 050 und in der französischen Patentschrift 7 017 514 beschrieben werden. Es ist auch möglich, die bei der technischen Isocyanatherstellung anfallenden Isocyanatgruppen aufweisenden Destillationsrückstände, gegebenenfalls gelöst in einem oder mehreren der vorgenannten Polyisocyanate, einzusetzen. Ferner ist es möglich, beliebige Mischungen der vorgenannten Polyisocyanate zu verwenden.

Geeignet sind auch Phosgenierungsprodukte von Kondensaten von Anilin und Aldehyden oder Ketonen, wie z.B. Acetaldehyd, Propionaldehyd, Butyraldehyd, Aceton, Methyläthylketon. Ferner geeignet sind die Phosgenierungsprodukte von Kondensaten von am Kern Alkyl substituierten Anilinen, insbesondere Toluidinen mit Aldehyden oder Ketonen, wie z.B. Formaldehyd, Acetaldehyd, Butyraldehyd, Aceton, Methyläthylketon.

Weiterhin geeignet sind Umsetzungsprodukte der genannten aromatischen Polyisocyanatgemische mit 0,2—50 Mol-% an Polyolen, vorausgesetzt, daß die Viskosität der so erhaltenen Umsetzungsprodukte 50 000 cP bei 25°C nicht überschreitet und der NCO-Gehalt der Umsetzungsprodukte mindestens 6 Gew.-% beträgt. Geeignete Polyole zur Modifizierung der Ausgangsmaterialien sind insbesondere die in der Polyurethan-Chemie bekannten Polyäther- und/oder Polyesterpolyole des Molekulargewichtsbereichs 200 bis 6000, vorzugsweise 300 bis 4000, sowie niedermolekulare Polyole des Molekulargewichtsbereichs 62 bis 200. Beispiele derartiger niedermolekularer Polyole sind Äthylenglykol, Propylenglykol, Glyzerin, Trimethylolpropan, 1,4,6-Hexantriol.

Besonders bevorzugte Isocyanatoaryl-sulfonsäuren sind die Sulfonierungsprodukte von 2,4-Toluylendiisocyanat sowie Gemischen aus 2,4- und 2,6-Toluylendiisocyanat, ferner Sulfonierungsprodukte der Di- bzw. Polyisocyanate, welche durch Phosgenierung von Anilin/Formaldehyd-Kondensaten erhalten werden. Diese Gemische enthalten insbesondere 4,4'-Diisocyanatodiphenylmethan und 2,4'-Diisocyanatodiphenylmethan sowie höherkernige Homologe dieser Produkte. Es ist grundsätzlich ohne Belang, mit welchen Sulfonierungsmitteln die Isocyanato-arylsulfonsäuren hergestellt worden sind. Geeignete Sulfonierungsmittel sind beispielsweise Schwefeltrioxid, Oleum, Schwefelsäure, Komplexe des Schwefeltrioxids mit Lewis-Basen, welche Sauerstoff-, Stickstoff-oder Phosphoratome enthalten. Es können jedoch auch andere bekannte Sulfonierungsmittel wie Chlorsulfonsäure und Acylsulfate, beispielsweise Acetylsulfat bzw. Umsetzungsprodukte von Säure-Anhydriden mit Schwefelsäure oder Oleum eingesetzt werden. Insbesondere zur Herstellung von nur partiell sulfonierten Isocyanaten spielen Nebenreaktionen, z.B. Harnstoff bzw. Biuretbildung oder die teilweise Umwandlung von Isocyanatgruppen in Carbamidsäurechlorid-Gruppen oder Acylamid-Gruppen im allgemeinen keine Rolle, so daß in diesen Fällen ohne weiteres beispielsweise Schwefelsäure, Chlorsulfonsäure oder Acetylsulfat eingesetzt werden können. Zur Herstellung von hochsulfonierten Polyisocyanaten wird dagegen Schwefeltrioxid bzw. seine Komplexe, beispielsweise gemäß DE—A 2 510 693, vorzugsweise eingesetzt. Hieraus folgt, daß insbesondere auch aromatische Polyisocyanatarylsulfonsäuren auf der Basis Toluylendiisocyanat bzw. Diphenylmethandiisocyanat bevorzugt werden, welche Harnstoff- bzw. Biuretgruppen enthalten.

Besonders bevorzugt sind Lösungen und Dispersionen von Isocyanato-aryl-sulfonsäuren in nichtsulfonierten flüssigen Polyisocyanaten. Solche Produkte werden beispielsweise bei teilweiser Sulfonierung aromatischer Polyisocyanate erhalten. Im allgemeinen erhält man bei der Teilsulfonierung von chemisch einheitlichen Diisocyanaten oder von binären Isomerengemischen Suspensionen, während bei der Teilsulfonierung von Mehrkomponenten-Gemischen homogene Lösungen entstehen. Für das erfindungsgemäße Verfahren ist es grundsätzlich ohne Belang, ob Lösungen oder Suspensionen eingesetzt werden. Ganz besonders bevorzugt sind teilsulfonierte Polyisocyanatgemische, wie sie durch Phosgenierung von Anilin-Formaldehyd-Kondensaten erhalten werden und in den deutschen Offenlegungsschriften 2 227 111, 2 359 614 und 2 359 615 beschrieben sind. Ebenfalls besonders bevorzugt sind Suspensionen von Diisocyanato-toluol-sulfonsäure-Dimeren sowie Diisocyanatodiphenylmethan-sulfonsäure-Dimeren in Diisocyanatotoluol bzw. Diisocyanatodiphenylmethan.

Die Herstellung der beim erfindungsgemäßen Verfahren einzusetzenden Isocyanatoarylsulfonsäuren bzw. ihrer Gemische mit nicht sulfonierten aromatischen Polyisocyanaten erfolgt nach den bekannten Verfahren des Standes der Technik bzw. in Analogie zu den bekannten Verfahren des Standes der Technik, wie er sich beispielsweise aus den bereits genannten Veröffentlichungen, oder aus US—PS 3 826 769 ergibt.

Beim erfindungsgemäßen Verfahren können auch Lösungen bzw. Suspensionen der oben beispielhaft genannten Isocyanatoarylsulfonsäuren in aliphatischen oder cycloaliphatischen Polyisocyanaten bzw. auch aromatischen, aliphatischen oder cycloaliphatischen Monoisocyanaten zum Einsatz gelangen. Beispiele solcher Isocyanate sind der US-Patentschrift 3 963 679 zu entnehmen.

Die Art und Mengenverhältnisse der beim erfindungsgemäßen Verfahren einzusetzenden Isocyanate bzw. deren Sulfonierungsgrad werden zweckmäßig so gewählt daß das Äquivalentverhältnis von (gegebenenfalls teilweise in dimerisierter Form vorliegenden) Isocyanatgruppen zu Sulfonsäuregruppen größer als 1.1 ist, und insbesondere zwischen 1.05 und 50:1, vorzugsweise zwischen 2:1 und 30:1, besonders bevorzugt zwischen 2:1 und 12:1 liegt.

Eine weitere Gruppe von erfindungsgemäß bevorzugten Isocyanatosulfonsäuren sind die in DE—A 2 615 876 beschriebenen aromatischen Mono-, Di- oder Polyisocyanate, welche mehr als eine Sulfonsäuregruppe (insbesondere 2 oder 3 Sulfonsäuregruppen) enthalten. In diesem Fall liegt das bevorzugte Verhältnis von Isocyanat- zu Sulfonsäuregruppen zwischen 0,5:1 und 1,2:1.

Beim erfindungsgemäßen Verfahren können als Oxiarane beliebige, mindestens eine Epoxidgruppe aufweisende, daneben gegebenenfalls noch mit Isocyanat- oder Hydroxylgruppen substituierte, ansonsten jedoch unter den Reaktionsbedingungen unter denen die Oxiran/Sulfonsäure-Addition erfolgt, weitgehend inerte organische Verbindungen eingesetzt werden. Vorzugsweise werden beim erfindungsgemäßen Verfahren dieser Definition entsprechende Monoepoxide des Molekulargewichtsbereichs 44—400 eingesetzt. Beispiele geeigneter Monoepoxide sind Äthylenoxid, Propylenoxid, Buten-1,2-oxid, Buten-2,3-oxid, 1,4-Dichlorbuten-2,3-oxid, Styroloxid, 1,1,1-Trichlorpropen-2,3-oxid, 1,1,1-Trichlorbuten-3,4-oxid, 1,4-Dibrombuten-2,3-oxid, Epichlorhydrin, Epibromhydrin, Glycid, Glycerin-mono-glycidyläther, Isobutenoxid, p-Glycidylstyrol, N-Glycidylcarbazol, Cyanäthylglydicyläther, Trichloräthylglycidyläther, Chloräthylglycidyläther, Bromäthylglycidyläther, Vinyloxiran, 3,4-Dichlorbuten-2-oxid, 2-(1-Chlorvinyl)-oxiran, 2-Chlor-2-vinyl-oxiran, 2,3-Epoxipropylphosphonsäurediäthylester, 3,4-Bis-hydroxy-buten-1,2-oxid, 2-Methyl-2-vinyl-oxiran, 2-(1-Methylvinyl-)oxiran. Gut geeignet sind auch Ester von Glycid mit Monocarbonsäuren, z.B. Glycidyl-acetat, Glycidyl-chloracetat, Glycidyl-dichloroacetat, Glycidyl-trichloroacetat, Glycidyl-bromacetat, Glycidyl-acrylat, Glycidylmethacrylat, Glycidyl-caproat, Glycidyl-octoat, Glycidyl-dodecanoat, Glycidyl-oleat, Glycidyl-stearat, sowie Äther des Glycids, z.B. mit Phenol und substituierten, insbesondere halogenierten Phenolen. Ebenfalls gut geeignet sind die Umsetzungsprodukte von Hydroxy-oxiranen, insbesondere von Glycid mit aliphatischen, cycloaliphatischen und aromatischen Mono- und Polyisocyanaten.

Zur Erhöhung des Molekulargewichts und der Funktionalität der erfindungsgemäß hergestellten Produkte können auch, gegebenenfalls anteilsweise, Di- und Polyepoxide eingesetzt werden, beispielsweise Epoxidationsprodukte von aliphatischen und cycloaliphatischen Diolefinen, epoxidierte Polybutadiene oder Butadien-Mischpolymerisate oder Polyglycidylester, wie sie beispielsweise durch Umsetzung einer Dicarbonsäure oder durch Umsetzung von Cyanursäure mit Epichlorhydriden oder Dichlorhydrin in Gegenwart von Alkali erhalten werden.

Polyglycidyläther, wie diejenigen, die man durch Veräthern eines zweiwertigen oder mehrwertigen Alkohols, eines Diphenols oder eines Polyphenols mit Epichlorhydrin oder Di-chlorhydrin in Gegenwart eines Alkalis erhält, werden vorzugsweise eingesetzt. Diese Verbindungen können sich von Glykolen, wie Äthylenglykol, Diäthylenglykol, Triäthylenglykol, 1,3-Propylenglykol, 1,4-Butandiol, 1,5-Pentandiol, 1,6-Hexandiol, 2,4,6-Hexantriol, Glycerin und insbesondere von Diphenolen oder Polyphenolen wie Resorcin, Brenzkatechin, Hydrochinon, Phenolphthalein, Phenol-formaldehyd-Kondensationsprodukten der Art der Novolake, 1,4-Di-hydroxynaphthalin, Dihydroxy-1,5-naphthalin, Bis-(hydroxy-4-phenyl)methan, ableiten. Insbesondere sind auch Epoxiharze geeignet, welche aus Polyphenolen hergestellt wurden und unter der Handelsbezeichnung Novolak—Harze vertrieben werden.

Weitere erfindungsgemäß geeignete Glycidäther sind den britischen Patenten 1 017 612, 1 024 288, 772 830 und 816 923 zu entnehmen. Weitere Epoxide, die erfindungsgemäß eingesetzt werden können, werden in Houben-Weyl, 1963, Band XIV/2, Seite 462 bis 538, beschrieben.

Eine Zusammenstellung technisch bedeutsamer Polyoxirane findet sich in H. Batzer und F. Lohse: Einführung in die makromolekulare Chemie, Hüthig & Wepf Verlag Basel, Heidelberg 1976 auf S. 44—53.

Als Oxetane kommen beim erfindungsgemäßen Verfahren beliebige, mindestens einen Oxetanring aufweisende, gegebenenfalls durch Isocyanat- oder Hydroxylgruppen substituierte, ansonsten jedoch unter den Reaktionbedingungen, unter denen die Oxetan/Sulfonsäure-Addition abläuft, weitgehend inerte organische Verbindungen in Betracht. Bevorzugte Oxetane sind dieser Definition entsprechende Monooxetane des Molekulargewichtsbereichs 58—400. Geeignete Oxetane können beispielsweise der deutschen Auslegeschrift 1 668 900, Kolonne 3 und 4, entnommen werden.

Selbstverständlich können auch die Oxetan-Analogen der weiter oben aufgeführten Glycid-Derivate eingesetzt werden, z.B. 3-Äthyl-3-acryloxy-oxetan, 3-Äthyl-3-methacryloxyoxetan, 3-Methyl-3-trichloracetoxy-oxetan, 3-Methyl-3-$\beta$-cyanäthoxymethyl-oxetan, 3-Äthyl-$\beta$-cyanäthoxymethyl-oxetan, 3-Äthyl-3-phenoxymethyl-oxetan.

Unter den erfindungsgemäß einsetzbaren Di- und Polyoxetanen sind von besonderer Bedeutung die Umsetzungsprodukte von 3-Alkyl-3-hydroxymethyl-oxetanen mit Di- und Polycarbonsäuren, sowie mit Di- und Polyisocyanaten. Auch die sich von aliphatischen, cycloaliphatischen und aromatischen Diolen und Polyolen ableitenden Di- und Polyäther der Hydroxy-oxetane sind sehr gut geeignet.

Die Oxirane sind als Ausgangsmaterialien im Rahmen des erfindungsgemäßen Verfahrens gegenüber den Oxetanen bevorzugt. Besonders bevorzugte Oxirane sind Äthylenoxid, Propylenoxid, Styroloxid, 1,1,1-Trichlorbuten-3,4-oxid und Epichlorhydrin. Bevorzugtes Oxetan ist 3-Hydroxymethyl-3-äthyl-oxetan.

Das Mengenverhältnis zwischen Polyhydroxyverbindungen und Isocyanatosulfonsäure wird meist so gewählt daß OH-funktionelle Produkte eines Molekulargewichts unter 12.000 und vorzugsweise unter 6,000 entstehen. Es wird also ein molarer Überschuß an hydroxyfunktionellen Komponenten eingesetzt, wobei auf eine NCO-Gruppe mindestens 1,5 OH-Gruppen und $SO_3H$-Gruppen entfallen sollen. Unter NCO-Gruppen sollen dabei nicht nur in freier Form vorliegende NCO-Gruppen verstanden werden sondenr auch in Form von Uretdion-Gruppen vorliegende dimerisierte NCO-Gruppen. Es ist besonders bevorzugt, die als Ausgangsmaterial verwendeten hydroxyfunktionellen Verbindungen nur anteilig mit Sulfonsäure-Gruppen zu modifizieren, wobei auf eine NCO-Gruppe bis zu 30 OH-Gruppen und $SO_3H$-Gruppen eingesetzt werden können. Bevorzugt ist ein Äquivalent-Verhältnis von OH-Gruppe zu NCO-Gruppe zwischen 2 und 20.

Die genannten Mengenverhältnisse gelten im Wesentlichen für Umsetzungen die unter Verwendung von Di- bzw. Polyisocyanaten durchgeführt werden und die unmittelbar zu durch Sulfonsäuregruppen modifizierten Polyhydroxyverbindungen führen, welche nach Umsetzung mit Oxiranen bzw. Oxetanen die erfindungsgemäßen Polyhydroxyverbindungen erhöhter Funktionalität ergeben.

Im Rahmen der vorliegenden Erfindung können jedoch auch Monoisocyanate, welche 1 bis 3 Sulfonsäuregruppen enthalten Verwendung finden. Diese Monoisocyanate können mit den Ausgans-Hydroxyverbindungen inmolar unterschüssigen Mengen aber auch in Äquivalenz eingesetzt werden, wobei im letztgenannten Fall alle OH-Funktionen mit Isocyanatgruppen umgesetzt werden, Man erhält Polysulfonsäuren, welche anschließend mit Oxiranen bzw. Oxetanen zu neuen Polyhydroxyverbindungen umgesetzt werden. Nach dieser Arbeitsweise können Produkte erhalten werden, deren OH-Funktionalität dieselbe ist, wie bei den eingesetzten Ausgangsverbindungen, deren Reaktivität jedoch verändert, beispielsweise verringert ist.

Die Umsetzung der Ausgangs-Hydroxyverbindungen mit den Sulfonsäuregruppen enthaltenden Isocyanaten erfolgt im Prinzip in bekannter Weise. Im allgemeinen werden die Hydroxyverbindungen vorgelegt und die Isocyanatkomponente unter Vermischen zugegeben. Ist das Isocyanat flüssig, wie es beispielsweise bei teilsulfonierten MDI-Typen der Fall ist, so kann die Vermischung der Komponenten und die anschließende Reaktion ohne Weiteres bei Raumtemperatur oder auch bei geringfügig erhöhter Temperatur stattfinden. Die Wahl der Temperatur hängt in diesem Fall ausschließlich von der Viskosität des Reaktionsgemisches und von der gewünschten Zeitdauer der Umsetzung ab. Bei Verwendung von festen Isocyanatoaryl-, Mono- oder Polysulfonsäuren entsteht bei der Vermischung primär eine Suspension und es ist zweckmäßig, die Umsetzung bei einer Temperatur vorzunehmen, bei der das feste Isocyanat rasch in Lösung geht.

Hierfür sind Temperaturen zwischen 40 und 180°C, insbesondere 60 und 120°C zweckmäßig. Insbesondere bei ausschließlichem Einsatz verhältnismäßig niedermolekularer Polyhydroxyverbinddungen werden Temperaturen über 120°C bis etwa 200°C bevorzugt, um ein Festwerden des Reaktionsansatzes während der Umsetzung zu vermeiden. Feste Isocyanatosulfonsäuren werden besonders bevorzugt in Form von Suspensionen, Pasten oder Feuchtpulvern, unter Verwendung inerter Lösungs mittel eingesetzt, wie dies in der DE-A-2 640 103 beschrieben ist.

Weiterhin können feste Isocyanatosulfonsäuren in der Form von Lösungen in organischen Lösungsmitteln eingesetzt werden, wobei als Lösungsmittel flüssige Ester einer anorganischen oder organischen Säure des Phosphors bevorzugt werden (DE-A-2 650 172).

Im übrigen können selbstverständlich beliebige inerte Lösungsmittel, wie Kohlenwasserstoffe, Halogenkohlenwasserstoffe, Äther, Ester und Ketone dem Reaktionsgemisch zugegeben werden. Bevorzugt ist indessen die Umsetzung in Abwesenheit von Lösungsmitteln bzw. mit den geringen Lösungsmittelmengen welche zum Anpasten oder Lösen fester Isocyanatosulfonsäuren Verwendung finden.

Die Umsetzung der eingeführten Sulfonsäuregruppen mit Oxiranen bzw Oxetanen kann entweder nach Umsetzung aller Isocyanatgruppen in einer 2. Reaktionsstufe oder aber auch gleichzeitig bzw. überlappend mit der Urethanisierungsreaktion vorgenommen werden. Eine Simultan-Reaktion kommt insbesondere dann in Betracht, wenn die OH-Gruppen der Ausgangskomponenten primär, die aus der Epoxydreaktion hervorgehenden OH-Gruppen dagegen sekundär sind. Unter diesen Voraussetzungen ist nur in untergeordnetem Maße mit einer Reaktion sekundärer OH-Gruppen mit Isocyanat-Gruppen zu rechnen.

Die erfindungsgemäßen Reaktionsprodukte lassen sich also grundsätzlich auch in einem Eintopf-Verfahren herstellen, wobei Hydroxyverbindungen, Isocyanatkomponente und Oxiran bzw. Oxetan gleichzeitig vermischt und miteinander umgesetzt werden. Dieses Verfahren kommt insbesondere auch bei schwerlöslichen Isocyanatosulfonsäuren in Betracht, da die Anwesenheit von Sauerstoff-Heterocyclen die Lösungsgeschwindigkeit erhöht.

Die beim erfindungsgemäßen Verfahren zum Einsatz gelangenden Oxirane bzw. Oxetane bzw. deren Mengen werden so gewählt, daß das Äquivalentverhältnis der Epoxid- bzw. Oxetan-Gruppen zu Sulfonsäuregruppen zwischen 0,2:1 und 5:1, vorzugsweise zwischen 0,6:1 und 2:1, liegt. Bei einem Äquivalentverhältnis von <1:1 werden die vorhandenen $SO_3H$-Gruppen nur teilweise verestert, so daß die erfindungsgemäßen Verfahrensprodukte noch freie Sulfonsäuregruppen aufweisen, so daß die durch diese Sulfonsäuregruppen bewirkte Hydrophilie der erfindungsgemäßen Verfahrensprodukte durch das genannte Äquivalentverhältnis innerhalb des Bereichs von 0,2:1 bis 1:1 variiert werden kann. Selbstverständlich kann die Epoxid- bzw. Oxetan-Komponente auch im Überschuß eingesetzt werden, um beispielsweise bei Verwendung von Mono-epoxiden bzw. Mono-oxetanen eine quantitative Veresterung der Sulfonsäuregruppen zu gewährleisten, oder um bei Verwendung von mehr als eine Epoxid- bzw. Oxetangruppe aufweisenden Verbindungen, freie Epoxid- bzw. Oxetan-Gruppen in die erfindungsgemäßen Verfahrensprodukte einzubauen. Insbesondere auf diese Weise eingebaute Epoxidgruppen können für Folgereaktionen, wie z.B. Trimerisierung der Isocanatgruppen, Oxazolidon-Bildung oder Amin-Vernetzung herangezogen werden.

Freie Sulfonsäuregruppen können auch ganz oder teilweise neutralisiert werden, beispielsweise mit tert. Aminen oder anorganischen Basen.

Ein gegebenenfalls eingesetzter Überschuß an Monoepoxid bzw. Monooxetan kann gewünschtenfalls nach Beendigung des erfindungsgemäßen Verfahrens destillativ aus dem erfindungsgemäßen Verfahrensprodukt entfernt werden.

Die Durchführung des erfindungsgemäßen Verfahrens ist denkbar einfach und erfolgt im allgemeinen im Temperaturbereich von 0—190°C, vorzugsweise 20—140°C.

Bei diskontinuierlicher Arbeitsweise wird das Gemisch bzw. Reaktionsprodukt aus Hydroxykomponente und Sulfonsäure-Gruppen enthaltendem Polyisocyanat vorzugsweise bei Raumtemperatur in einem Rührgefäß vorgelegt und das Epoxid bzw. Oxetan eingerührt. Die Reaktion setzt im allgemeinen sofort unter Selbsterwärmung ein. Wenn der Anteil an Sulfonsäure-Gruppen mehr als ca. 10% beträgt, kann es zweckmäßig sein, die Reaktion bei tieferen Temperaturen, z.B. zwischen 0 und 20°C durchzuführen und gegebenenfalls unter Kühlung zu arbeiten. Eine solche Maßnahme ist jedoch im Regelfall nicht erforderlich, da eine Erwärmung des Reaktionsgemisches auf beispielsweise 140°C oder auch darüber nicht von Nachteil ist. Wenn auf eine rasche Reaktion innerhalb kurzer Zeit Wert gelegt wird, sowie im Falle der Verwendung von bei Raumtemperatur flüssigen Epoxiden und Oxetanen bzw. viskosen Isocyanaten, kann es zweckmäßig sein, die Reaktion bei erhöhter Temperatur vorzunehmen, beispielsweise zwischen 40—140°C, in Sonderfällen kann die Temperatur bis ungefähr 190°C betragen.

Gasförmige Epoxide werden zweckmäßigerweise unter Rühren eingeleitet. Die Reaktion wird vorzugsweise lösungsmittelfrei durchgeführt, jedoch kann selbstverständlich auch in Anwesenheit inerter Lösungsmittel, wie z.B. Dichloräthan, Chloroform, Tetrachloräthan, Trichlorfluormethan, Aceton, Toluol, Chlorbenzol gearbeitet werden.

Eine besonders starke Funktionalitätserhöhung läßt sich durch Verwendung von Di- oder Polyoxiranen bzw. den entsprechenden Oxetanen erreichen, insbesondere, wenn ungefähr in Äquivalenz zu den vorhandenen Sulfonsäuregruppen gearbeitet wird. Es ist bei einer solchen Arbeitsweise leicht möglich OH-Funktionalitäten von 4 bis 8 zu erreichen. Es ist jedoch auch möglich eine Funktionalität unter 4 einzustellen, wenn Monoisocyanatoarylmonosulfonsäuren und/oder monofunktionelle Alkohole zumindest anteilig eingesetzt werden.

Zur Erhöhung der Funktionalität ganz besonders geeignete Verbindungen sind auch Oxirane und Oxetane mit OH-Gruppen, z.B. Glycid, 3-Methyl-3-hydroxymethyl-oxetan, 3-Äthyl-3-hydroxymethyl-oxetan. Je nach gewünschter Reaktivität der OH-funktionellen Kurzkette wird man Oxiranen oder Oxetanen den Vorzug geben. Wahrend Oxetane in der Regel primäre OH-Gruppen liefern, führt die Verwendung von Oxiranen meist zu sekundaren oder sogar tertiären OH-Gruppen. Lediglich Äthylenoxid gibt eine primäre OH-Gruppe, Glycid führt gleichzeitig eine primäre und eine sekundäre OH-Gruppe innerhalb einer Kurzkette ein.

Werden Di- oder Polyoxirane in molar überschüssigen Mengen eingesetzt, so daß nur ein Teil der Epoxygruppen mit Sulfonsäuregruppen reagieren, so werden Polyhydroxyverbindungen erhalten, welche noch freie Epoxygruppen aufweisen, welche entweder mit beispielsweise Carbonsäuren oder Carbonsäureanhydriden zur Reaktion gebracht werden können, oder aber als Reaktionsharze im Rahmen der Epoxydchemie eingesetzt werden können.

Die erfindungsgemäßen Verfahrensprodukte sind wertvolle Ausgangsmaterialien zur Herstellung von Polyurethankunststoffen nach dem Isocyanat-Polyadditionsverfahren. Sie eignen sich z.B. zur Herstellung von kompakten oder zelligen Elastomeren, Weichschäumen, Halbhartschäumen und Hartschäumen, insbesondere dann, wenn hohe Anforderungen an die Vernetzungsdichte, das Brandverhalten oder die Abbaubarkeit gestellt werden. So eignen sich die erfindungsgemäßen Polyhydroxyverbindungen beispielsweise zur Herstellung von Polstermaterialien, Matratzen, elastischen Unterlagen, Autositzen, Dampfungsmaterialien, Stoßabsorbern, Konstruktionswerstoffen, schalldämmenden Isolierungen, feuchtigkeitsaufnehmenden Materialien, z.B. im Hygienesektor, zur Herstellung von Substraten zur Pflanzenaufzucht, sowie für den Wärme- und Kälteschutz. Ganz besonders geeignet sind die

erfindungsgemäßen Polyhydroxyverbindungen zur Herstellung anorganisch-organischer Kunststoff beispielsweise in Analogie zu den in DBP 2 310 559, DE-A-2 227 147, 2 359 608 beschriebenen Verfahrensweisen, sowie für Oberflächenbeschichtungen, Imprägnierungen und Verklebungen geeignet.

Ein besonderer Vorteil der erfindungsgemäßen Hydroxy-Verbindungen ist ihre erhöhte Polarität. Dadurch sind diese Produkte im Gegensatz beispielsweise zu reinen Polypropylenglykoläthern mit niedermolekularen Glykolen wie Äthylenglykol, Diäthylenglykol, 1,4-Butandiol, Glyzerin, gut verträglich. Mischungen sind homogen und damit lagerstabil. Zur Herstellung von Polyadditionsprodukten mit günstigem Brandverhalten ist die Umsetzung der Polyhydroxyverbindungen gemäß vorliegender Erfindung mit Sulfonsäureester-Gruppen aufweisenden Polyisocyanaten besonders günstig.

Derartige, Sulfonsäure- oder Sulfonsäreester-Gruppen aufweisende Polyisocyanate sind infolge ihrer hohen Polarität mit hydrophoben, langkettigen Polyäthern häufig wenig vertraglich so daß Entmischungseffekte auftreten, die unter Umstanden eine Polyadditionsreaktion unmöglich machen. Werden solche Polyäther gemäß vorliegender Erfindung mit Sulfonsäureester-Gruppen modifiziert, so ist meist eine gute Verträglichkeit mit Sulfonsäure- oder Sulfonsäureester-Gruppen aufweisenden Polyisocyanaten gegeben.

## Beispiel 1

(a) Herstellung der Isocyanatoarylsulfonsäure

1914 g (11 Mol) Toluylendiisocyant (Isomerengemisch 2,4: 2,6 = 80:20) werden bei 23—30°C im Verlauf von ca. 20 Stunden unter Rühren mit 335 g (4,2 Mol) Schwefeltrioxid umgesetzt, wobei eine dickflüssige Suspension der dimeren Toluylendiisocyanat-monosulfonsäure im Toluylendiisocyanat ensteht. Das Schwefeltrioxid wird mittels eines schwachen Stickstoffstroms aus erwärmten 65% igem Oleum freigesetzt und gasförmig mit Stickstoff verdünnt auf die Oberfläche des gerührten Isocyanates aufgeleitet. Die erhaltene Suspension wird mit 500 ml Toluol verdünnt, abgesaugt, und der feste Rückstand 2 mal mit 500 ml Toluol angeschlämmt und abgesaugt. Das toluol-feuchte Produkt wird abgefüllt. Ausbeute 1285 g, Gehalt an Toluol 23%, Trockensubstanz 990 g, entsprechend 93% der Theorie.

Das Produkt ist ein schwach feuchtes Pulver, das sich ohne zu stauben sehr gut handhaben läßt. Es ist leicht ab- und umzufüllen, backt nicht zusammen und klebt nicht am Spatel.

b) Herstellung eines durch Sulfonsäuregruppen modifizierten Polyätherdiols:

200 g (0,1 Mol) eines linearen difunktionellen Polypropylenglykols vom Molekulargewicht 2000 werden bei 50—60°C mit 16,5 g (0,05 Mol) des nach a) hergestellten Produkts verrührt. Nach 5 Stunden ist eine homogene Schmelze entstanden, die frei von NCO-Gruppen ist. Nach Zugabe von 4,6 g (0,05 Mol) Epichlorhydrin wird noch 1 Stunde bei 60°C nachgerührt. Viskosität bei 25°C: 2 800 mPas.

Durchschnittsfunktionalität des Produkts: 3.

## Beispiel 2

Es wird wie im Beispiel 1 verfahren, anstelle des Epichlorhydrins werden jedoch 8,5 g (0,025) Bisphenol-A-diglycidyläther zugesetzt. Viskosität bei 25°C: 50 000 mPas.

Durchschnittsfunktionalität des Produkts: 6

## Beispiel 3

240 g (0,12 Mol) eines linearen Polypropylenglykols vom Molekulargewicht 2000 werden bei 30°C mit 33 g (0,1 Mol) des nach Beispiel 1 a) hergestellten Produkts verrührt. Man erhält eine weiße Suspension, die bei 60°C allmählich in eine klare Flüssigkeit übergeht. Nach Einrühren von 9,25 g (0,1 Mol) Epichlorhydrin wird noch 30 Minuten bei 40—50°C nachgerührt.

Viskosität bei 25°C: 25 000 mPas.

## Beispiel 4

300 g (0,1 Mol) eines trifunktionellen auf Trimethylolpropan gestarteten Polypropylenglykols vom Molekulargewicht 3000 werden bei Raumtemperatur mit 16,5 g (0,05 Mol) des nach 1 a) hergestellten Produkts verrührt. Nach Aufheizen auf 60°C entsteht allmählich eine klare Flüssigkeit, in welche 4,6 g (0,05 Mol) Epichlorhydrin eingerührt wird.

Viskosität bei 25°C: 3400 mPas

## Beispiel 5

In Abänderung von Beispiel 4 wird unmittelbar nach dem Isocyanat das Epichlorhydrin eingerührt. Es wird 90 Minuten bei Raumtemperatur gerührt, wobei sich das Isocyanat teilweise löst. Anschließend wird auf 60°C erwärmt und 7 Stunden bei dieser Temperatur gerührt. Man erhält eine klare hellbraune Flüssigkeit, die keine NCO-Gruppen mehr enthält. Viskosität bei 25°C: 4500 mPas. Hellbraune, klare Flüssigkeit.

Mittlere Funktionalität: 5

Beispiel 6

Es wird wie in Beispiel 5 verfahren, jedoch unter Verwendung von 3,7 g (0,05 Mol) Glycid anstelle des Epichlorhydrins. Hellgelbe trübe Flüssigkeit.

Viskositätbei 25°C: 4500 mPas

Mittlere Funktionalität: 6

Beispiel 7

Es wird wie in Beispiel 5 verfahren, jedoch unter Verwendung von 5,8 g 3-Äthyl-3 hydroxymethyloxetan anstelle des Epichlorhydrins. Es wurde 8 Stunden bei 60°C, 6 Stunden bei 80°C, 6 Stunden bei 95°C und 2 Stunden bei 120°C gerührt. 4 g ungelöstes Isocyanat wurden abfiltriert Braune, grün fluoreszierende Flüssigkeit.

Viskosität bei 25°C: 4000 mPas

Mittlere Funktionalität: 6

Beispiel 8

200 g (0,2 Mol) eines linearen difunktionellen Polypropylenglykols vom Molekulargewicht 1000 werden bei 70°C mit 33 g (0,1 Mol) des nach Beispiel 1 a hergestellten Produkts, welches mit 30 g Toluol angeschlämmt wird, verrührt. Nach 1 Stunde ist eine klare Schmelze entstanden, nach 4 Stunden sind keine NCO-Gruppen mehr vorhanden (IR-Spektrum). Toluol wird bei 70°C i. Vak. abgezogen und das Reaktionsprodukt bei 25°C mit 7,4 g (0,1 Mol) Glycid verrührt.

Viskosität bei 25°C: 11,000 mPas

Durchschnittsfunktionalität des Produkts: 4

OH-Zahl: 106

Säurezahl (Ester-Spaltung): 14

Beispiel 9

Es wird analog Beispiel 8 gearbeitet, jedoch unter Verwendung von 123 g (0,2 Mol) eines linearen Polyäthylenglykols vom Molekulargewicht 615.

Viskosität bei 25°C: 30 000 mPas

Durchschnittsfunktionalität: 4

OH-Zahl: 136

Säurezahl (Ester-Spaltung): 26

Beispiel 10

Es wird analog Beispiel 8 gearbeitet, jedoch unter Verwendung von 80 g (0,2 Mol) Octaäthylenglykol.

Viskosität bei 25°C: 120 000 mPas

Durchschnittsfunktionalität: 4

OH-Zahl: 168

Säurezahl (Ester-Spaltung): 42

Beispiel 11

20 g des nach Beispiel 8 erhaltenen Produkts und 8 g des nachfolgend als Polyisocyanat A beschriebenen Produkts werden gemischt, wobei eine homogene weißgelbe Paste entsteht. Nach 4 Stunden ist eine plastische hochmolekulare Masse entstanden, die noch weiteren 2 Stunden zu einem Elastomeren vernetzt.

Wird derselbe Versuch mit 0,3 g Zinndioctoat durchgeführt, so ist die Masse bereits 2 Stunden nach Vermischen vernetzt. Das vernetzte Elastomer ist homogen, klebfrei und von guter Zugfestigkeit.

Vergleichsversuch

Beispiel 11 wird wiederhalt mit 20 g des im Beispiel 8 als Ausgangsmaterial verwendeten Polypropylenglykols vom MG 1000 und 8 g des nachfolgend als Polyisocyanat A beschriebenen Produktes. Man erhält ein zweiphasiges Gemisch, dessen dunkle in sich heterogene Isocyanatphase sich schlammig absetzt. Innerhalb von 5 Stunden wird die Mischung mehrfach durch Rühren vermischt, jedoch tritt stets nach wenigen Minuten wieder Phasentrennung ein. Nach 8 Stunden ist die Mischung noch flüssig.

Wird derselbe Versuch mit 0,3 g Zinn-dioctoat durchgeführt, so entsteht unmittelbar nach dem Vermischen in stark exothermer Reaktion ein inhomogenes festes Produkt mit krümeligem Aussehen. Das Produkt ist klebrig und ohne Festigkeit.

Polyisocyanat A

Vom rohen Phosgenierungsprodukt eines Anilin-Formaldehyd-Kondensats wird so viel Diisocyanatodiphenylmethan abdestilliert, daß der Destillationsrückstand bei 25°C eine Viskosität von 50 mPas aufweist (2-Kern-Anteil: 68 Gew.-%; 3-Kern-Anteil: 16 Gew.-%; Anteil an höherkernigem Polyisocyanat: 16 Gew.-%; NCO-Gehalt: 32 Gew.-%). Auf die Oberfläche von 3800 g dieses Produkts wird

ein Gemisch aus Schwefeltrioxyd und Stickstoff aufgeleitet und zwar so lange, bis 102 g Schwefeltrioxyd von dem Isocyanatgemisch aufgenommen worden sind. Das erhaltene Produkt hat eine Viskosität von 120 mPas und einen Schwefelgehalt von 1,05%.

1850 g dieses sulfonierten Polyisocyanats werden bei Raumtemperatur innerhalb 30 Minuten mit 35,2 g Propylenoxid versetzt. Anschließend wird 4 Stunden bei 25—30°C nachgerührt. Nach 20 Tagen hat das erhaltene mit Sulfonsäureester-Gruppen modifiziert Polyisocyanat eine Viskosität von 490 cP und einen Schwefelgehalt von 1,03%.

## Patentansprüche

1. Mindestens zwei Hydroxylgruppen und mindestens eine Sulfonsäureester-Gruppe aufweisende Verbindungen vom durchschnittlichen Molekulargewicht 300 bis 12000, in denen mindestens eine Hydroxylgruppe in Form eines Urethan-aryl-sulfonsäure-hydroxyalkylestersvorliegt.

2. Verbindungen gemäß Anspruch 1, gekennzeichnet durch mindestens eine OH-funktionelle Langkette, welche 6—400 Kettenglieder enthält und mindestens eine, über einen Sulfonsäureester-Rest mit einer Verzweigungsstelle verbundene OH-funktionelle Kurzkette, welche 2 bis 3 Kettenglieder enthält, sowie mindestens einen mindestens trifunktionellen Arylrest als Verzweigungsstelle.

3. Verbindungen gemäß Anspruch 2, gekennzeichnet durch mindestens eine OH-funktionelle Langkette welche 20—300 Kettenglieder enthält.

4. Verfahren zur Herstellung von mindestens zwei Hydroxylgruppen und mindestens eine Sulfonsäureestergruppe aufweisenden Verbindungen vom durchschnittlichen Molekulargewicht 300 bis 12000, in denen mindestens eine Hydroxylgruppe in Form eines Urethan-aryl-sulfonsäure-hydroxyalkylesters vorliegt, dadurch gekennzeichnet, daß mindestens zwei Hydroxylgruppen aufweisende Verbindungen vom Molekulargewicht 62 bis 10000 bei 0–190°C mit aromatischen Isocyanatosulfonsäuren und anschließend mit Oxiranen und/oder Oxetanen umgesetzt werden, wobei das Äquivalent-Verhältnis der Gesamtmenge an Isocyanatgruppen (einschließlich der gegebenenfalls in dimerisierter Form vorhandenen Isocyanatgruppen) zu Sulfonsäuregruppen 0,5 bis 50, das Äquivalent-Verhältnis aus der Summe der Hydroxyl-Gruppen der mindestens zwei Hydroxylgruppen aufweisenden Verbindungen und der Sulfonsäuregruppen zu NCO-Gruppen 1,5 bis 30 und das Äquivalent-Verhältnis der Oxiran- bzw. Oxetan-Gruppen zu Sulfonsäuregruppen 0,2 bis 5 beträgt.

5. Verwendung der Verbindungen gemäß Anspruch 1 als Reaktionskomponente für Polyisocyanate zur Herstellung von Polyadditionsprodukten und/oder Polykondensationsprodukten.

## Claims

1. Compounds containing at least two hydroxyl groups and at least one sulphonic acid ester group and having an average molecular weight of from 300 to 12,000, in which at least one hydroxyl group is present in the form of a urethane aryl sulphonic acid hydroxyalkyl ester.

2. Compounds according to Claim 1, characterised by at least one OH-functional long chain, which contains from 6 to 400 chain members and at least one OH-functional short chain which contains 2 to 3 chain members which is attached to a branching point through a sulphonic acid ester residue and at least one at least trifunctional aryl radical as the branching point.

3. Compounds according to Claim 2, characterised by at least one OH-functional long chain containing from 20 to 300 chain members.

4. A process for producing compounds containing at least two hydroxyl groups and at least one sulphonic acid ester group and having an average molecular weight of from 300 to 12,000, in which at least one hydroxyl group is present in the form of a urethane aryl sulphonic acid hydroxyalkyl ester, characterised in that compounds containing at least two hydroxyl groups and having a molecular weight of from 62 to 10,000 are reacted at from 0 to 190°C with aromatic isocyanatosulphonic acids and then with oxiranes and/or oxetanes, the equivalent ratio of the total quantity of isocyanate groups (including the isocyanate groups optionally present in dimerised form) to sulphonic acid groups amounting to from 0.5:1 to 50:1, the equivalent ratio of the sum of the hydroxyl groups in the compounds containing at least two hydroxyl groups and the sulphonic acid groups to NCO groups amounting to from 1.5:1 to 30:1 and the equivalent ratio of the oxirane or oxetane groups to sulphonic acid groups amounting to from 0.2:1 to 5:1.

5. The use of the compounds according to Claim 1 as reaction component for polyisocyanates for the production of polyaddition products and/or polycondensation products.

## Revendications

1. Composés comportant au moins deux groupes hydroxy et au moins un groupe d'ester d'acide sulfonique, ces composés ayant un poids moléculaire moyen de 300 à 12.000, tandis qu'ils comportent

au moins un groupe hydroxy sous forme d'un ester hydroxyalkylique d'acide uréthane-aryl-sulfonique.

2. Composés suivant la revendication 1, caractérisés par au moins une longue chaîne à fonctionnalité OH contenant 6 à 400 chaînons, et par au moins une courte chaîne à fonctionnalité OH reliée à un point de ramification par un radical d'ester d'acide sulfonique et contenant 2 ou 3 chaînons, ainsi que par au moins un groupe aryle au moins trifonctionnel comme point de ramification.

3. Composés suivant la revendication 2, caractérisés en ce qu'ils comportent au moins une longe chaîne à fonctionnalité OH contenant 20 à 300 chaînons.

4. Procédé de préparation de composés comportant au moins deux groupes hydroxy et au moins un groupe d'ester d'acide sulfonique, ces composés ayant un poids moléculaire moyen de 300 à 12.000, tandis qu'ils comportent au moins un groupe hydroxy sous forme d'un ester hydroxyalkylique d'acide uréthane-aryl-sulfonique, caractérisé en ce qu'on fait réagir des composés comportant au moins deux groupes hydroxy et ayant un poids moléculaire de 62 à 10.000, à une température de 0 à 190°C, avec des acides isocyanato-sulfoniques aromatiques, puis avec des oxirannes et/ou oxétannes, le rapport équivalent entre la quantité totale de groupes isocyanates (y compris les groupes isocyanates éventuellement présents sous forme dimérisée) et les groupes d'acides sulfoniques étant de 0,5 à 50, le rapport équivalent entre la somme des groupes hydroxy des composés comportant au moins deux groupes hydroxy, ainsi que des groupes d'acides sulfoniques, et les groupes NCO étant de 1,5 à 30, tandis que le rapport équivalent entre les groupes oxirannes ou oxétannes et les groupes d'acides sulfoniques est de 0,2 à 5.

5. Utilisation des composés suivant la revendication 1 comme composants réactionnels pour des polyisocyanates en vue de préparer des produits de polyaddition et/ou des produits de polycondensation.